Europäisches Patentamt

European Patent Office

Office européen des brevets

(19)

(11) Veröffentlichungsnummer: **0 287 029**
**A2**

# EUROPÄISCHE PATENTANMELDUNG

(21) Anmeldenummer: 88105802.8

(22) Anmeldetag: 12.04.88

(51) Int. Cl.4: **A61K 31/70**

---

Die Bezeichnung der Erfindung wurde geändert (Richtlinien für die Prüfung im EPA, A-III, 7.3).

(30) Priorität: 14.04.87 DE 3712622

(43) Veröffentlichungstag der Anmeldung:
19.10.88 Patentblatt 88/42

(84) Benannte Vertragsstaaten:
**AT BE CH DE ES FR GB GR IT LI LU NL SE**

(71) Anmelder: **Pulverer, Gerhard, Prof. Dr. med.**
**Mohnweg 25**
**D-5000 Köln 40(DE)**

Anmelder: **Oette, Kurt, Prof. Dr. med.**
**Braunstrasse 39**
**D-5000 Köln 41(DE)**

Anmelder: **Uhlenbruck, Gerd, Prof. Dr. med.**
**Gleueler Strasse 308**
**D-5000 Köln 41(DE)**

(72) Erfinder: **Pulverer, Gerhard, Prof. Dr. med.**
**Mohnweg 25**
**D-5000 Köln 40(DE)**
Erfinder: **Oette, Kurt, Prof. Dr. med.**
**Braunstrasse 39**
**D-5000 Köln 41(DE)**
Erfinder: **Uhlenbruck, Gerd, Prof. Dr. med.**
**Gleueler Strasse 308**
**D-5000 Köln 41(DE)**

(74) Vertreter: **Werner, Hans-Karsten, Dr. et al**
**Deichmannhaus am Hauptbahnhof**
**D-5000 Köln 1(DE)**

---

(54) Verwendung von Monosacchariden und/oder diese Monosaccharide enthaltenden Glykokonjugaten zur Behandlung von Infektionen durch Krankheitserreger.

(57) Infektionen von Krankheitserregern können verhindert und/oder behandelt werden durch Applikation von für Lektine der Organzellen oder der Krankheitserreger spezifischen Monosacchariden und/oder diese Monosaccharide enthaltenden Glykokonjugaten.

EP 0 287 029 A2

## Verwendung von Monosacchariden und/oder diese Monosaccharide enthaltenden Glykokonjugaten

Infektionen durch Krankheitserreger sind meistens organspezifisch, ohne daß man bisher eine Erklärung für diese Organspezifität hatte. Neuere Untersuchungen der Erfinder, aber auch anderer Forschungsgruppen deuteten darauf hin, daß Lektine und Zuckerstrukturen hierbei eine Rolle spielen.

Lektine sind hochspezifische zuckerbindende Moleküle, welche zunächst nur in Pflanzen, später aber auch in nahezu allen anderen Lebewesen bis hin zu Vertebraten gefunden wurden. Die Lektine dienen anscheinend in der Hauptsache der Erkennung von Zuckerstrukturen an Zelloberflächen oder in löslichen Glykokonjugaten.

Die Erfinder haben bereits festgestellt, daß Organzell-Lektine für die spezifisch organotrope Metastasierung von Tumoren verantwortlich sind. Sie haben daher in der europäischen Patentanmeldung 87 105 548.9 Mittel zur Verhinderung von Metastasen maligner Tumore vorgeschlagen, enthaltend die für Organzell-Lektine spezifischen Monosaccharide und/oder diese Monosaccharide enthaltenden Glykokonjugate.

Weitere intensive Untersuchungen haben jetzt gezeigt, daß auch für die organspezifischen Infektionen durch Krankheitserreger Lektine verantwortlich sind, und zwar teilweise Lektine der Organzellen und teilweise Lektine der Krankheitserreger selbst. Schließlich wurde jetzt gefunden, daß die Infektion durch Krankheitserreger verhindert und/oder behandelt werden kann, wenn man diese Lektine mit den für sie spezifischen Monosacchariden und/oder endständig diese Monosaccharide enthaltenden Glykokonjugaten absättigt.

Von besonderer Bedeutung scheinen dabei die β-D-Galactose und/oder endständige β-D-Galactose enthaltenden Glykokonjugate zu sein. Weitere bedeutungsvolle Monosaccharide sind die Mannose sowie endständige oder mittelständige Mannose enthaltende Glykokonjugate sowie L-Fukose, N-Acetylglukosamin, N-Acetyl-Galactosamin, N-Acetyl-Neuraminsäuren und Neuraminsäure-haltige Derivate. Die Glykokonjugate sind somit Substanzen, die das spezifische Monosaccharid endständig oder mittelständig gebunden haben an ein pharmakologisch inertes Trägermolekül. Dieses Trägermolekül soll zumindest nicht selbst toxisch oder statisch gegen die Krankheitserreger wirksam sein. Typische Trägermoleküle sind somit im einfachsten Fall andere Zucker, so daß erfindungsgemäß außer den spezifischen Monosacchariden selbst auch diese enthaltende Disaccharide, Trisaccharide und Oligosaccharide eingesetzt werden können. Vorzugsweise sollen die Glykokonjugate nicht oder nur schwach immunogen sein, um nicht zu Nebenwirkungen zu führen oder durch die Immunantwort unwirksam gemacht zu werden.

In den die spezifischen Monosaccharide enthaltenden Glykokonjugaten sollten sie vorzugsweise endständig gebunden sein. Es hat sich jedoch gezeigt, daß auch mittelständig gebundene Monosaccharide wirksam sind. Hierbei ist jedoch noch nicht geklärt, ob in diesen Glykokonjugaten der eine oder der andere Rest so leicht abgespalten wird, daß in vivo rasch Glykokonjugate mit einem endständigen spezifischen Monosaccharidrest entstehen.

Typische Krankheitserreger, deren Infektionen und Behandlung erfindungsgemäß möglich ist, sind vor allem Bakterien, aber auch Viren, Pilze und Parasiten, welche Erkrankungen wie Malaria, Toxoplasmose etc. auslösen.

Es handelt sich somit insbesondere um sich intrazellulär vermehrende Parasiten, die jedoch auch meist eine hohe Organspezifität aufweisen.

So konnte jetzt gezeigt werden, daß beispielsweise Infektionen durch Streptococcus pneumoniae durch Applikation von N-Acetyl-D-glucosamin/D-galactose (GlcNAc-Gal) verhindert werden können, so daß die für Lungengewebe spezifischen Bakterien nicht mehr in der Lage sind, sich in Lungengewebe festzusetzen und dort ihre gefährliche Wirkung zu entfalten. In gleicher Weise wurde gefunden, daß durch Applikation von N-Acetyl-neuraminsäure (NANA) die organspezifische Ansiedlung von Pseudomonas aeruginosa verhindert werden kann. Weitere vorläufige Ergebnisse deuten darauf hin, daß durch Applikation von β-D-Galactose und/oder β-D-Galactose enthaltenden Glykokonjugaten, wie Arabinogalactan, die erste Entwicklungsphase des Erregers der Malaria in der Leber unterbunden werden kann. Erfindungsgemäß wäre es dann möglich, vorbeugend gegen Infektion durch Malaria β-B-Galactose und/oder β-D-Galactose enthaltende Glykokonjugate einzusetzen anstelle des heute überwiegend eingesetzten Chinins.

Obwohl die für Lektin der Organzellen oder der Krankheitserreger spezifischen Monosaccharide und/oder diese Monosaccharide enthaltenden Glykokonjugate in erster Linie die organspezifische Ansiedlung der Krankheitserreger unterbinden, erscheint es in einigen Fällen durchaus möglich, auch die bereits - schon organspezifisch gebundenen Krankheitserreger durch Verdrängungsreaktionen mit Hilfe der für die Lektine spezifischen Monosaccharide und/oder diese Monosaccharide enthaltenden Glykokonjugate wieder von den Organen abzulösen. Zumindest wird durch die Applikation der Monosaccharide bei bereits erfolgter Infektion das Fortschreiten der Infektion durch weitere Krankheitserreger unterbunden, so daß auch noch nach erfolgter Infektion eine Behandlung möglich ist.

Gegenstand der vorliegenden Erfindung ist somit die Verwendung von für Lektine der Organzellen oder der Krankheitserreger spezifischen Monosacchariden und oder diese Monosaccharide enthaltenden Glykokonjugaten zur Verhinderung und oder Behandlung von Infektionen durch diese Krankheitserreger.

Weiterhin betrifft die Erfindung die Verwendung von für Lektine der Organzellen oder der Krankheitserreger spezifischen Monosacchariden und oder diese Monosaccharide enthaltenden Glykokonjugaten zur Herstellung von Mitteln zur Verhinderung und oder Behandlung von Infektionen durch diese Krankheitserreger.

Schließlich betrifft die Erfindung Verfahren zur Verhinderung und oder Behandlung von Infektionen von Krankheitserregern durch Applikation von für Lektine der Organzellen oder der Krankheitserreger spezifischen Monosacchariden und oder diese Monosaccharide enthaltenden Glykokonjugaten.

Bevorzugte Verwendungen sind insbesondere die bereits oben erwähnten Beispiele, nämlich die Verwendung von N-Acetyl-D-glucosamin D-galactose (GlcNAc-Gal) gegen Infektionen von Streptococcus pneumoniae sowie die Verwendung von N-Acetyl-neuraminsäure (NANA) gegen Infektionen von Pseudomonas aeruginosa. Schließlich ist es die Verwendung von $\beta$-D-Galactose und oder $\beta$-D-Galactose enthaltenden Glykokonjugaten zur Verhinderung von Infektionen von Malaria.

Die Tatsache, daß die meisten Infektionen durch Krankheitserreger organspezifisch sind, spricht jedoch dafür, daß auch hierbei für Lektine der Organzellen oder der Krankheitserreger spezifische Monosaccharide und oder diese Monosaccharide enthaltende Glykokonjugate geeignet sind, die Infektionen zu verhindern und oder zu behandeln.

Die Monosaccharide und oder diese Monosaccharide enthaltende Glykokonjugate sind größtenteils bereits bekannt und ausgesprochen gut verträglich. Die neue Therapie kann somit relativ einfach und problemlos durchgeführt werden. Die Wirkstoffe können sowohl enteral als auch parenteral appliziert werden. Sie werden in bekannter Weise metabolisiert oder aus dem Körper ausgeschieden. Der relativ hohe Serumspiegel an den Monosacchariden und oder den diese Monosaccharide enthaltenden Glykokonjugaten reicht offensichtlich aus, die entscheidenden Lektine so lange zu blockieren, bis die Infektionsgefahr verringert oder beseitigt ist. Dies kann beispielsweise dadurch erklärt werden, daß die in den Körper eingedrungenen Krankheitserreger nach einer ausreichend langen Zeit von dem körpereigenen Abwehrsystem erkannt und unschädlich gemacht werden, ehe sie sich organspezifisch ansiedeln können.

Es ist einleuchtend, daß die erfindungsgemäß verwendeten Mittel wesentlich einfacher, preiswerter und sicherer zu handhaben sind als die bisher verwendeten Therapeutika.

Aus umfangreichen Tierexperimenten kann geschlossen werden, daß bei Applikation von 50 bis 200 g Monosacchariden und oder diese Monosaccharide enthaltenden Glykokonjugaten pro Tag Infektionen erfolgreich verhindert werden können und auch schon eingetretene Infektionen erfolgreich behandelt werden können.

Typische Beispiele für Monosaccharide, die für die Lektine der Ziel-Organzellen spezifisch sind, sind N-Acetyl-D-glucosamin D-Galactose gegen Streptococcus pneumoniae und N-Acetylneuraminsäure gegen Pseudomonas aeruginosa. Die folgenden Festsetz-Versuche wurden durchgeführt mit Streptococcus pneumoniae Pn 629, Typ 14, und Pseudomonas aeruginosa ATCC 27853. Die die Hemmung betreffenden Versuche wurden mit N-Acetyl-D-glucosamin und N-Acetylneuraminsäure durchgeführt, um die Beteiligung der N-Acetyl-D-glucosamin-und N-Acetylneuraminsäure-spezifischen Lektine an dem Vorgang des Festsetzens dieser Mikroorganismen aufzuzeigen. Hämagglutinationstests zur Spezifizierung der Oberflächenlektine von Pseudomonas aeruginosa Stamm ATCC 27853 zeigten, daß N-Acetylneuraminsäure befähigt ist, die Hämagglutination von Kaninchen-Erythrozyten zu hemmen. D-Mannose-oder D-Galactose-spezifische Lektine konnten in diesem Stamm nicht gezeigt werden.

Pseudomonas aeruginosa ATCC 27853 wurde in Mueller-Hinton-Brühe (Oxoid, UK) kultiviert, und Streptococcus pneumoniae Pn 629, Typ 14, in Herzinfusions-Blut-Brühe (Difco, USA). Nach der Kultur (24 h bei 37 °C) wurden die Bakterien-Suspensionen zentrifugiert, in PBS (Flow Laboratories, FRG) re-suspendiert, zweimal gewaschen und auf eine Trübung des McFarland-Standards Nr. 5 eingestellt. Human-Nieren-Proben wurden aus dem makroskopisch und histologisch normalen Pol einer Niere mit einem renalen Adenokarzinom am entgegengesetzten Pol erhalten. Lungengewebe wurde nach einer Lobektomie wegen einer neoplastischen Erkrankung erhalten, das histologisch frei von transformierten Zellen war. Unmittelbar nach dem Entfernen wurden die Gewebe-Proben mit PBS gewaschen, in OCT-Verbindung (Miles Laboratories, USA) eingebettet, in flüssigem Stickstoff schockgefroren und bis zum Gebrauch bei -70 °C aufbewahrt. Für die Festsetz-Analysen wurden Kryotom-Schnitte (LKB-Kryostat, Bromma, Schweden) der in OCT-Verbindung eingebetteten, in Stickstoff gefrorenen Schnitte von Lunge und Niere präpariert, wie kürzlich beschrieben wurde. Kurz gesagt, die Organ-Proben wurden auf Glas-Objektträgern befestigt und (unter mäßiger Bewegung) 30 min bei 4 °C entweder mit 30 $\mu$l Pseudomonas aeruginosa ATCC 27853 (1 $\times$

$10^9$ ml), suspendiert in PBS oder in Lösungen von PBS und Kohlenhydrat (D-Mannose, N-Acetyl-D-glucosamin, D-Xylose, N-Acetylneuraminsäure), oder mit 30 $\mu$l Streptococcus pneumoniae Pn 629 (1 × $10^9$ ml), suspendiert in PBS oder den PBS-Kohlenhydrat-Lösungen wie oben, inkubiert. Nach intensivem Waschen (45 s) in eiskaltem PBS erfolgten die Fixierung nach Netland und Zetter, Gram-oder Methylenblau-Färbung und die mikroskopische Untersuchung und mikrophotographische Aufnahme.

Zur Demonstration des lektinabhängigen Mechanismus der Bakterien-Festsetzung wurden Kryotom-Schnitte der menschlichen Lunge und der menschlichen Niere mit in PBS (oder PBS-Kohlenhydrat-Lösungen) suspendiertem Pseudomonas aeruginosa ATCC 27853 und Streptococcus pneumoniae Pn 629, Typ 14, inkubiert. Sowohl die Schnitte der menschlichen Lunge als auch der menschlichen Niere unterstützten die Festsetzung von Pseudomonas aeruginosa. Ein Festsetzen von Streptococcus pneumoniae war jedoch nachweisbar nur für die Lungen-Zellen, nicht aber für die Nieren-Zellen. Diese experimentelle Beobachtung steht im Einklang mit der klinischen Erfahrung bei Infektionen durch Pseudomonas aeruginosa, die sowohl die Lunge als auch die Harnwege befallen, und Pneumokokken-Infektionen, die in erster Linie die Lunge betreffen. Eine rezeptorvermittelte Organotropie infektiöser Mittel wird durch diese Befunde nahegelegt.

Die Zugabe der lektinspezifischen und aufgrunddessen lektinblockierenden Kohlenhydrate N-Acetyl-D-glucosamin (5 mg/ml PBS für Streptococcus pneumoniae) und N-Acetylneuraminsäure (1,25 mg/ml PBS für Pseudomonas aeruginosa) hemmte die Festsetzung dieser Mikroorganismen an den Organzellen vollständig. Lösungen von D-Mannose und D-Xylose (jeweils 5 mg/ml PBS) zeigten keinerlei Hemmwirkung, was die Spezifität dieses lektinvermittelten Erkennungsvorgangs demonstriert. Diese in vitro mit Human-Gewebe erhaltenen stimmen mit den bei Balb/c-Mäusen erhaltenen Ergebnissen überein. Da ein Festsetzen von Streptococcus pneumoniae und Pseudomonas aeruginosa an Organzellen verhindert wird, wenn bakterielle Lektin-Rezeptoren mittels kompetitiver, spezifischer Kohlenhydrate (Tabelle 1) blockiert werden, sind diese Substanzen für die Verhütung oder Behandlung von Infektionskrankheiten wertvoll.

T a b e l l e   1

Lektin-induzierte Festsetzung von Pseudomonas aeruginosa ATCC 27853 und Streptococcus pneumoniae Pn 629, Typ 14, (beide jeweils 1 x $10^9$/ml, suspendiert in PBS oder in PBS, das unspezifische Kohlenhydrate enthält) und ihre spezifische Hemmung durch bakteri-elle Lektin-Blockierung (N-Acetyl-D-glucosamin, GlcNAc, und N-Acetylneuraminsäure, NANA)

| | Festsetzung von P. aeruginosa | | Festsetzung von S. pneumoniae | |
| | Lunge | Niere | Lunge | Niere |
|---|---|---|---|---|
| PBS | ++ | ++ | ++ | (+) |
| PBS + GlcNAc[a] | ++ | ++ | - | - |
| PBS + NANA[b] | - | - | ++ | (+) |
| PBS + D-Mannose[a] | ++ | ++ | ++ | (+) |
| PBS + D-Xylose[a] | ++ | ++ | ++ | (+) |

[a]   5 mg, gelöst in 1 ml PBS.

[b]   1,25 mg, gelöst in 1 ml PBS.

++   stark;   (+) schwach;   - keine Festsetzung.

(PBS = phosphatgepufferte Kochsalzlösung ["Saline"]).

## Ansprüche

1. Verwendung von für Lektine der Organzellen oder der Krankheitserreger spezifischen Monosacchariden und/oder diese Monosaccharide enthaltenden Glykokonjugaten zur Herstellung von Mitteln zur Verhinderung und/oder Behandlung von Infektionen durch diese Krankheitserreger.

2. Verwendung gemäß Anspruch 1, dadurch gekennzeichnet, daß N-Acetyl-D-glucosamin/D-galactose (GlcNAc-Gal) eingesetzt wird zur Herstellung von Mitteln gegen Streptococcus pneumoniae.

3. Verwendung gemäß Anspruch 1, dadurch gekennzeichnet, daß N-Acetyl-neuraminsäure (NANA) eingesetzt wird zur Herstellung von Mitteln gegen Infektionen von Pseudomonas aeruginosa.

4. Verwendung gemäß Anspruch 1, dadurch gekennzeichnet, daß β-D-Galactose und/oder β-D-Galactose enthaltende Glykokonjugate eingesetzt werden zur Herstellung von Mitteln gegen Infektionen von Malaria.